# EUROPEAN PATENT SPECIFICATION

(11) **EP 4 331 640 B1**
(45) Date of publication and mention of the grant of the patent: **15.04.2026**
(21) Application number: 23194222.8
(22) Date of filing: 30.08.2023
(51) Int. Cl.: A61M 1/34

(54) **DIALYSIS APPARATUS AND METHOD FOR CHECKING CONNECTION OF REPLENISHER PASSAGE**
DIALYSEVORRICHTUNG UND VERFAHREN ZUR ÜBERPRÜFUNG DER VERBINDUNG EINES REGENERATORDURCHGANGS
APPAREIL DE DIALYSE ET PROCÉDÉ DE VÉRIFICATION DE LA CONNEXION D'UN PASSAGE DE RÉGÉNÉRATEUR

(30) Priority: 02.09.2022 JP 2022140313
(43) Date of publication of application: 06.03.2024
(73) Proprietor: Shibuya Corporation, Kanazawa-shi, Ishikawa 920-8681 (JP)
(72) Inventor: MATSUBARA, Yosuke, Kanazawa-shi, 920-8681 (JP); MISHIMA, Takashi, Kanazawa-shi, 920-8681 (JP); MATSUZAKI, Kosho, Kanazawa-shi, 920-8681 (JP); NAKAMURA, Yuki, Kanazawa-shi, 920-8681 (JP)
(74) Representative: Mewburn Ellis LLP

(56) References cited:
- WO-A1-2018/190433

## Description

### BACKGROUND OF THE INVENTION

### Field of the Invention

The present invention relates to a dialysis apparatus and a method for checking connection of a replenisher passage, and more particularly to a dialysis apparatus including a replenisher passage for performing fluid replenishment by supplying a dialysate in a dialysate circuit to a blood circuit, and a method for checking connection of a replenisher passage for checking a connection position of the above-described replenisher passage.

### Description of the Related Art

A dialysis apparatus used in hemodialysis or the like includes a dialyzer that performs hemodialysis, a dialysate supply passage that supplies a fresh dialysate to the above-described dialyzer, a dialysate collection passage that collects a used dialysate that has passed through the dialyzer, and a blood circuit including an arterial side passage that supplies blood to the above-described dialyzer and a venous side passage that discharges blood from the dialyzer.

Further, dialysis apparatuses are known in each of which a replenisher passage is provided between the above-described dialysate supply passage and the above-described blood circuit to perform fluid replenishment to a patient during dialysis treatment, and among them, there is also known a dialysis apparatus in which the above-described replenisher passage is selectively connectable to an arterial side passage or a venous side passage (Japanese Patent No. 6900756).

Here, the case in which fluid replenishment is performed by connecting the above-described replenisher passage to the arterial side passage that is at an upstream side from the dialyzer in the blood circuit is called pre-replenishment (pre-dilution method), whereas the case where fluid replenishment is performed by connecting the above-described replenisher passage to a venous side passage that is at a downstream side from the dialyzer is called post-replenishment (post-dilution method), and selection of the post-replenishment or pre-replenishment is judged by a physician according to the physical condition of a patient.

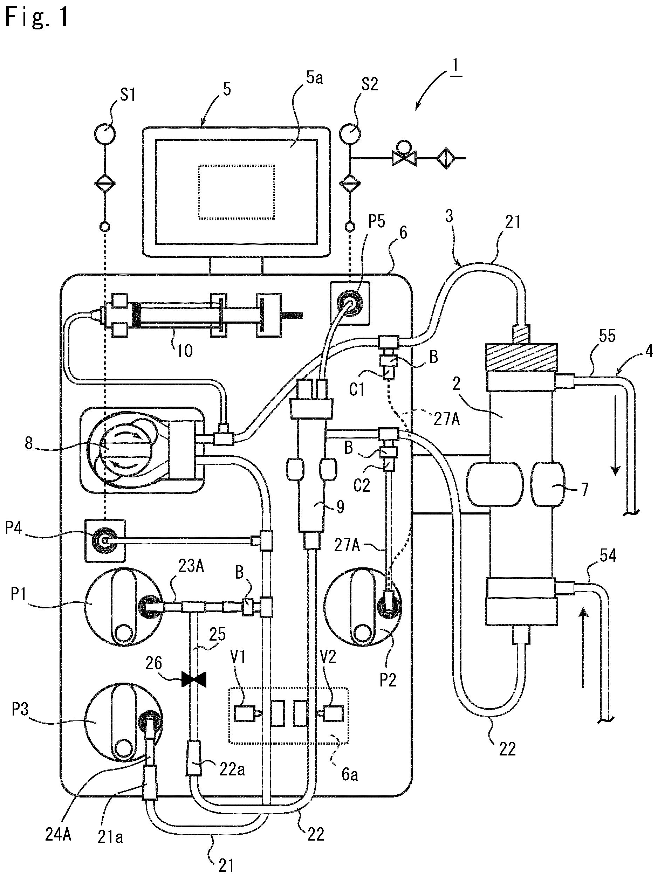

However, because the dialysis apparatus comprises many pipes as shown in FIG. 1, a connection error may occur when a healthcare worker connects the replenisher passage to the arterial side passage or the venous side passage as directed by a physician.

Thus, in Japanese Patent No. 6900756, it is determined whether the replenisher passage is connected to the arterial side passage or connected to the venous side passage by causing the dialysate to flow to the blood circuit that is not filled with a liquid, and detecting whether or not air bubbles are detected by an air bubble detector.

Here, in Japanese Patent No. 6900756 described above, detection of air bubbles is performed by connecting one end side of the arterial side passage and the other end side of the venous side passage, and causing a liquid to flow into the blood circuit from a state where the liquid is not filled.

Therefore, if a connection error of the replenisher passage is determined, and the determination method according to Japanese Patent No. 6900756 is used again after the replenisher passage is reconnected, there arises the problem that an operation is complicated because the blood circuit is already filled with the liquid and the liquid has to be removed from the blood circuit.

Document WO2018190433 is also considered as part of the prior art.

In addition, it is difficult to completely remove the liquid from the blood circuit, and air bubbles may be contained in the liquid remaining in the blood circuit, so that when the above-described determination method is executed again, there is a risk of occurrence of false detection.

In view of the problems as above, the present invention provides a dialysis apparatus and a method for checking connection of a replenisher passage, which can determine a connection destination of a replenisher passage more easily and reliably.

### SUMMARY OF THE INVENTION

A dialysis apparatus according to the invention of claim 1 is a dialysis apparatus comprising a dialyzer that performs hemodialysis, a dialysate supply passage that supplies a fresh dialysate to the dialyzer, a dialysate collection passage that collects a used dialysate that passes through the dialyzer, a blood circuit comprising an arterial side passage that supplies blood to the dialyzer and a venous side passage that discharges blood from the dialyzer, a replenisher passage provided between the dialysate supply passage and the blood circuit, and liquid delivery device that delivers the dialysate of the dialysate supply passage to the blood circuit via the replenisher passage,

the replenisher passage being provided to be selectively connectable to the arterial side passage or the venous side passage of the blood circuit, the dialysis apparatus being characterized by comprising
a supply side pressure sensor that measures a pressure of a liquid flowing through the dialysate supply passage, a collection side pressure sensor that measures a pressure of a liquid flowing through the dialysate collection passage, and a connection passage in which one end communicates with the arterial side passage or the venous side passage of the blood circuit, and the other end communicates with the dialysate collection passage, and characterized in that
determination device is further provided, the determination device determining whether the replenisher passage is connected to the arterial side passage, or the venous side passage based on a differential pressure between a pressure measured by the supply side pressure sensor and a pressure measured by the collection side pressure sensor, in a state where the liquid delivery device causes a liquid to flow into the blood circuit from the replenisher passage, and the liquid flows through the dialysate collection passage via the connection passage from the arterial side passage or the venous side passage.

A method for checking connection of a replenisher passage before performing dialysis treatment and not including dialysis treatment according to the invention of claim 4 is a method for checking connection of a replenisher passage, including a dialyzer that performs hemodialysis, a dialysate supply passage that supplies a fresh dialysate to the dialyzer, a dialysate collection passage that collects a used dialysate that passes through the dialyzer, a blood circuit comprising an arterial side passage that supplies blood to the dialyzer and a venous side passage that discharges blood from the dialyzer, a replenisher passage provided between the dialysate supply passage and the blood circuit, and liquid delivery device that delivers the dialysate in the dialysate supply passage to the blood circuit via the replenisher passage, and determining whether the replenisher passage is connected to the arterial side passage, or the venous side passage in the blood circuit, the method being characterized in that
a supply side pressure sensor that measures a pressure in the dialysate supply passage, and a collection side pressure sensor that measures a pressure in the dialysate collection passage are provided,
in a state where either one of the arterial side passage or the venous side passage of the blood circuit is caused to communicate with the dialysate collection passage, the liquid delivery device causes a liquid to flow into the blood circuit from the replenisher passage, and the liquid is caused to flow to the dialysate collection passage from the arterial side passage or the venous side passage, and
it is determined whether a connection destination of the replenisher passage is the arterial side passage, or the venous side passage based on a differential pressure between a pressure measured by the supply side pressure sensor and a pressure measured by the collection side pressure sensor.

According to the above-described invention, when the liquid delivery device causes the dialysate to flow into the blood circuit from the above-described replenisher passage, the dialysate flows through the dialysate collection passage via the above-described connection passage from the arterial side passage or the venous side passage.

At this time, there arises the case where the liquid passes through the dialyzer provided in the blood circuit, or the case where the liquid does not pass through the dialyzer, according to the position where the replenisher passage is connected, and when the liquid passes through the dialyzer, a pressure loss is generated by the liquid passing through countless hollow fibers provided in the dialyzer.

When the liquid passes through the dialyzer, the differential pressure occurs between the pressure measured by the above-described supply side pressure sensor and the pressure measured by the collection side pressure sensor, and therefore, it is possible to determine whether the above-described replenisher passage is connected to the above-described arterial side passage, or venous side passage, depending on the magnitude of the differential pressure like this.

### BRIEF DESCRIPTION OF THE DRAWINGS

FIG. 1 is a front view of a dialysis apparatus according to a first embodiment;
FIG. 2 is a circuit diagram of the dialysis apparatus, which is a circuit diagram when performing post-replenishment;
FIG. 3 is a circuit diagram of the dialysis apparatus, which is a circuit diagram when performing post-replenishment;
FIG. 4 is a circuit diagram of the dialysis apparatus, which is a circuit diagram when performing pre-replenishment; and
FIG. 5 is a circuit diagram of the dialysis apparatus, which is a circuit diagram when performing pre-replenishment.

### DETAILED DESCRIPTION OF THE PREFERRED EMBODIMENT

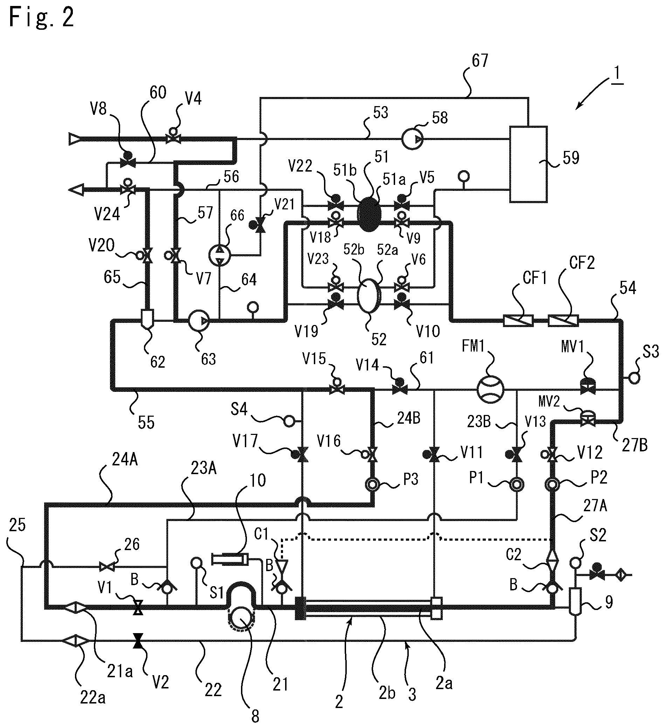

Explaining an illustrated embodiment hereinafter, FIG. 1 shows a front view of a dialysis apparatus 1 according to the present embodiment, and FIGS. 2 to 5 show circuit diagrams of the dialysis apparatus 1, and particularly show states during priming operation in the above-described dialysis apparatus 1.

The above-described priming operation refers to an operation of connecting a dialyzer 2 and a blood circuit 3 to the above-described dialysis apparatus 1 before performing dialysis treatment and filling the dialyzer 2 and the blood circuit 3 with a dialysate.

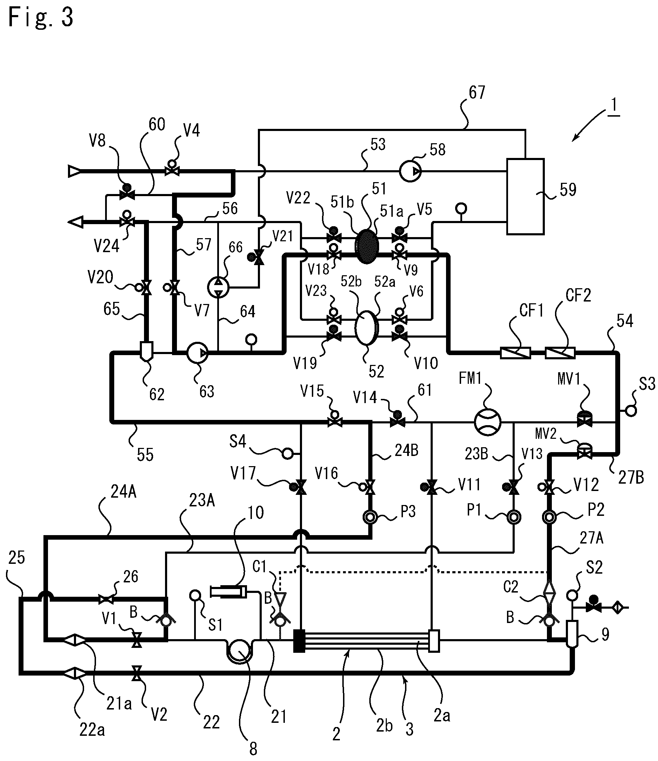

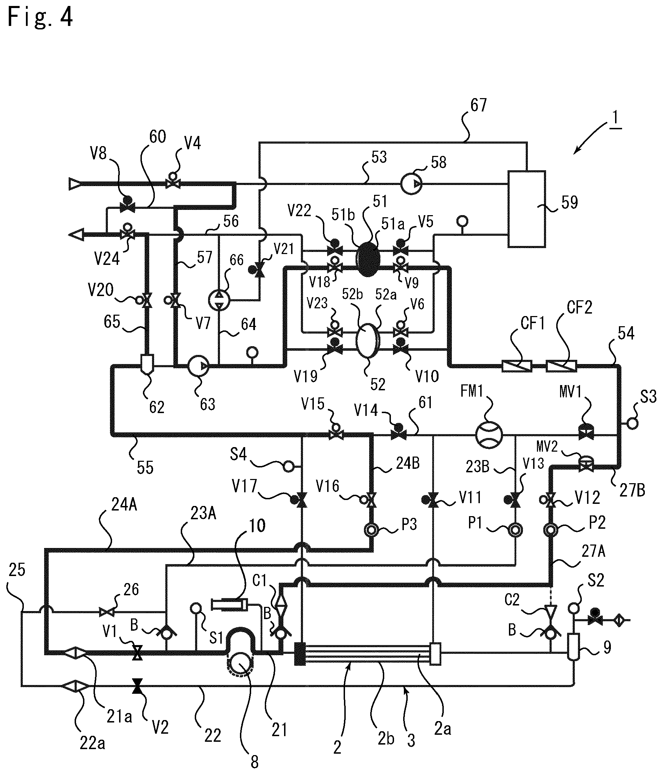

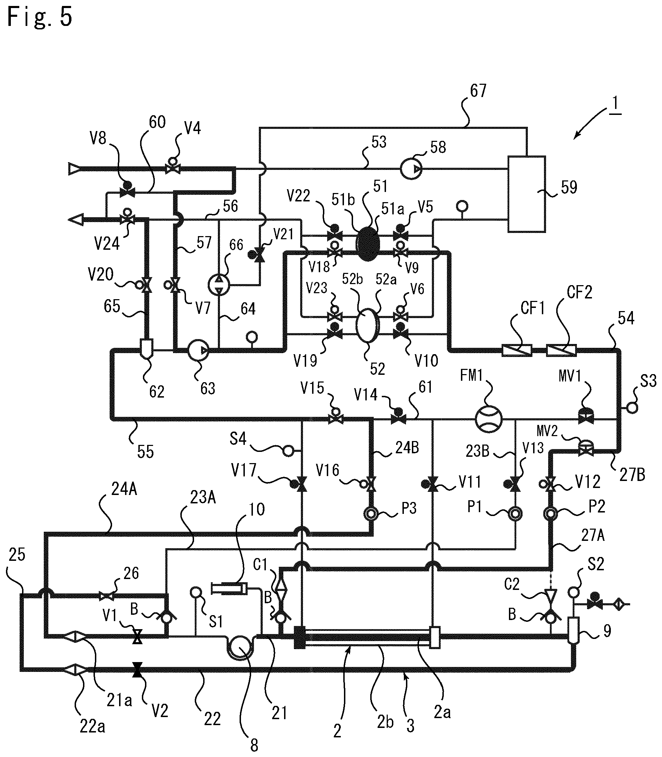

Further, in the dialysis apparatus 1 of the present embodiment, it is possible to perform replenishment by a dialysate to a patient under dialysis treatment, and as the replenishment like this, it is possible to selectively perform post-replenishment that is performed by connecting a replenisher passage to a venous side passage of a blood circuit 3 shown in FIG. 2 and FIG. 3, and pre-replenishment that is performed by connecting the replenisher passage to an arterial side passage shown in FIG. 4 and FIG. 5.

The post-replenishment and pre-replenishment are directed by a physician according to a physical condition or the like of a patient, and because a healthcare worker performs connection of the replenisher passage, it has been necessary to prevent a connection error.

Thus, in the dialysis apparatus 1 of the present embodiment, it is made possible to confirm whether or not connection is performed as directed by a physician by determining whether the replenisher passage is connected to the venous side passage, or the arterial side passage of the blood circuit 3 in parallel with the priming operation.

The above-described dialysis apparatus 1 includes the dialyzer 2 that performs hemodialysis, the blood circuit 3 that causes blood to flow through the dialyzer 2, and a dialysate circuit 4 that causes a dialysate to flow through the dialyzer 2, and is controlled by control device 5 including a calculator such as a microcomputer, or a personal computer.

In FIG. 1, most of the above-described dialyzer 2 and blood circuit 3 are provided to be exposed on a front surface of a body portion 6 composing the dialysis apparatus 1, and on the other hand, most of the dialysate circuit 4 is accommodated inside the dialysis apparatus 1.

At an upper part of the above-described body portion 6, a touch panel 5a that composes the above-described control device 5 and is to display a condition and the like of a patient under dialysis treatment and perform necessary operations is provided, and on a side surface, a dialyzer holder 7 that holds the above-described dialyzer 2 is provided.

Further, on a front surface of the body portion 6, a blood pump 8 for delivering blood of the blood circuit 3 is provided, and a drip chamber 9 composing the blood circuit 3, and a syringe 10 for drug injection are held in addition.

Further, on the front surface of the above-described body portion 6, a first to a third connection ports P1 to P3 that are provided to connect the blood circuit 3 and the dialysate circuit 4, and first and second sensor ports P4 and P5 for connecting some pipes of the blood circuit 3 to measure a pressure of the blood circuit 3 are provided.

For these connection ports P, it is possible to use the configuration described in Japanese Patent No. 5920575, for example, and each one comprises a connector provided at an end portion of a pipe composing the dialysate circuit 4, and a lid member rotatably provided on the front surface of the body portion 6.

By the configuration like this, for example, during priming operation, it is possible to rotate the above-described lid member to expose the above-described connector to outside and connect the pipe of the blood circuit 3 thereto, and during dialysis operation, it is possible to remove the pipe and cover the above-described connector so that the connector is not exposed to outside.

The above-described dialyzer 2 includes countless hollow fibers inside a cylindrical portion of resin, an inside of the hollow fibers forms a blood chamber 2a through which blood flows, and an outside of the hollow fibers forms a dialysate container 2b through which the dialysate flows.

Caps are provided on both end portions of the above-described dialyzer 2, the above-described blood circuit 3 is connected to a center of each of the caps to communicate with the above-described blood chamber 2a, and the above-described dialysate circuit 4 is connected to a side portion of the cap to communicate with the above-described dialysate container 2b.

As shown in FIG. 1, the dialyzer 2 is held by the above-described dialyzer holder 7 so that the above-described caps are positioned up and down, and during dialysis treatment, blood flows through the above-described blood chamber 2a from a top toward a bottom in the drawing (to the right from the left illustrated in FIG. 2), whereas the dialysate flows through the dialysate container 2b from the bottom toward the top in the drawing (to the left from the right illustrated in FIG. 2).

The above-described blood circuit 3 includes an arterial side passage 21 for delivering blood to the blood chamber 2a of the dialyzer 2 from an artery of a patient, and a venous side passage 22 for returning blood to a vein of the patient from the blood chamber 2a. In FIG. 1, the arterial side passage 21 is connected to a top portion of the above-described dialyzer 2, and the venous side passage 22 is connected to a bottom portion of the dialyzer 2.

In FIG. 2, a connector 21a to which a puncture needle is fitted during treatment is provided at an end portion of the above-described arterial side passage 21, and in order from a position adjacent to the connector 21a, an arterial side clamp V1, a blood return passage 23A for returning blood to a patient after dialysis treatment, an arterial side pressure sensor S1 that measures a pressure of the arterial side passage 21, the syringe 10 for charging a drug to the blood circuit 3, and a pre-replenishment connector C1 for connecting the replenisher passage when performing pre-replenishment are provided.

In the dialysis apparatus 1 under priming, one end of a first priming pipe 24A as a connection passage is connected to the connector 21a of the above-described arterial side passage 21, and the other end of the first priming pipe 24A is connected to the above-described third connection port P3 provided at an end portion of a connection passage 24B composing the above-described dialysate circuit 4.

The above-described arterial side clamp V1 is accommodated in a case 6a provided on the front surface of the body portion 6, and opening and closing of the arterial side passage 21 are automatically performed by drive device that is not illustrated and controlled by the control device 5.

The above-described blood return passage 23A is used when returning the blood in the blood circuit 3 to a patient after dialysis treatment, a check valve B is provided in a vicinity of a branched portion from the arterial side passage 21, which is a base portion, and a tip end is connected to the first connection port P1 provided at an end portion of a blood return passage 23B composing the above-described dialysate circuit 4.

A second priming pipe 25 as a connection passage that is used during priming is branched and provided in a middle of the blood return passage 23A, and is connected to a connector 22a at an end portion of the above-described venous side passage 22 at a time of priming. Further, the second priming pipe 25 is closable by a manual clamp 26.

In FIG. 1, the arterial side pressure sensor S1 includes the first sensor port P4 on the front surface of the body portion 6, and a pipe branched from the arterial side passage 21 is used by being coupled to the first sensor port P4.

The above-described blood pump 8 is composed of a so-called tube pump, and presses a pipe composing the arterial side passage 21 with rotation of a rotor, thereby delivering a liquid in the pipe in such a manner as to push out the liquid.

Note that when the arterial side passage 21 is filled with the dialysate during priming shown in FIG. 2 and FIG. 4, the arterial side passage 21 is not fitted to the blood pump 8, and flow of the liquid in the arterial side passage 21 is not inhibited.

A drug is contained in the above-described syringe 10, a plunger is advanced by pressing device controlled by the control device 5, and at the time of dialysis treatment, the drug is automatically supplied to the blood circuit 3 from the syringe 10.

As shown in FIG. 4 and FIG. 5, a replenisher passage 27A is connected to the above-described pre-replenishment connector C1 when performing pre-replenishment, and connectors are provided at both ends of the replenisher passage 27A.

One connector of the replenisher passage 27A is connected to the second connection port P2 provided at an end portion of a replenisher passage 27B composing the above-described dialysate circuit 4, and the connector at the other end is connected to either the above-described pre-replenishment connector C1 or a post-replenishment connector C2 provided at the venous side passage 22 and described later.

The above-described pre-replenishment connector C1 is provided in a position branched from the above-described arterial side passage 21, a check valve B is provided between the pre-replenishment connector C1 and the above-described arterial side passage 21 so as to prevent backflow from the blood circuit 3.

The above-described venous side passage 22 is connected to a lower end portion of the dialyzer 2 in FIG. 1, and a connector 22a to which a puncture needle is fitted at the time of dialysis treatment is provided at an end portion. In the venous side passage 22, in order from a dialyzer 2 side, the post-replenishment connector C2 to which the above-described replenisher passage 27A is connected when performing post-replenishment, the above-described drip chamber 9, and a venous side clamp V2 are provided.

As shown in FIG. 2 and FIG. 3, the replenisher passage 27A is connected to the above-described post-replenishment connector C2 when performing post-replenishment.

Further, the above-described post-replenishment connector C2 has a same structure as the above-described pre-replenishment connector C1, and backflow from the blood circuit 3 is prevented by the check valve B provided between the post-replenishment connector C2 and the above-described venous side passage 22.

As shown in FIG. 1, the above-described drip chamber 9 includes a cylindrical container and a cap that are held at the front surface of the body portion 6 of the dialysis apparatus 1, and a venous side pressure sensor S2 that measures a pressure of the venous side passage 22 is connected to the above-described cap.

In FIG. 1, the above-described venous side pressure sensor S2 includes a second sensor port P5 on the front surface of the body portion 6, and a pipe branched from the venous side passage 22 is used by being coupled to the second sensor port P5.

The above-described dialysate circuit 4 includes first and second dialysate chambers 51 and 52 inside of which supply chambers 51a and 52a and collection chambers 51b and 52b are formed by a diaphragm, a liquid supply passage 53 that supplies a dialysate to the above-described supply chambers 51a and 52a, a dialysate supply passage 54 that supplies a fresh dialysate to the dialyzer 2 from the supply chambers 51a and 52a, a dialysate collection passage 55 that collects a used dialysate from the dialyzer 2 to the collection chambers 51b and 52b, and a liquid discharge passage 56 that discharges the used dialysate from the above-described collection chambers 51b and 52b.

In the above-described liquid supply passage 53, an end portion at an upstream side is connected to a supply water source that is not illustrated and supplies clean water, and in order from an upstream side of a supply water source side, a water supply valve V4 that is controlled by the control device 5, a first bypass passage 57 communicating with the above-described liquid discharge passage 56, a liquid supply pump 58 that delivers the clean water of the supply water source, and undiluted dialysate supply device 59 that supplies an undiluted solution of dialysate are provided.

An end portion at a downstream side of the liquid supply passage 53 is branched toward the above-described first and second dialysate chambers 51 and 52, and liquid supply valves V5 and V6 that are controlled by the control device 5 are respectively provided at branched passages.

The above-described first bypass passage 57 is provided to be branched at an upstream side of the above-described liquid supply pump 58, and the other end thereof is connected to the above-described liquid discharge passage 56. Further, a seventh on-off valve V7 that is controlled by the control device 5, and a second bypass passage 60 that is branched at an upstream side of the seventh on-off valve V7 are provided in a middle of the first bypass passage 57.

The other end of the above-described second bypass passage 60 is connected to the above-described liquid discharge passage 56, and the second bypass passage 60 is provided with an eighth on-off valve V8 that is controlled by the control device 5.

The above-described undiluted dialysate supply device 59 includes undiluted solution tanks that contain an A undiluted solution and a B undiluted solution that are raw materials of the dialysate though not illustrated, and a liquid delivery pump that supplies a predetermined amount of each of the A undiluted solution and the B undiluted solution in the undiluted solution tanks.

The A undiluted solution and the B undiluted solution that are delivered by the above-described liquid delivery pump flow through the liquid supply passage 53 with clean water that is delivered by the above-described liquid supply pump 58, and flow into the supply chambers 51a and 52a of the above-described first and second dialysate chambers 51 and 52.

These A undiluted solution and B undiluted solution are agitated with the clean water inside of the above-described supply chambers 51a and 52a and inside of two filters CF1 and CF2 or the like described later, and thereby a dialysate of a predetermined concentration is prepared.

An upstream portion of the above-described dialysate supply passage 54 is branched in two directions, and connected to the supply chambers 51a and 52a of the above-described first and second dialysate chambers 51 and 52 respectively, and an end portion at a downstream side is connected to an end portion at a lower side of the dialyzer 2 in FIG. 1 to communicate with the above-described dialysate container 2b.

Further, at the above-described branched portions in the dialysate supply passage 54, supply valves V9 and V10 are respectively provided, and in a position at a downstream side from the branched portions, in order from an upstream side, the two filters CF1 and CF2 that adsorb endotoxin and the like in the dialysate, a supply side pressure sensor S3 that measures a pressure of the dialysate that flows through the dialysate supply passage 54, first flow rate restriction device MV1, a flowmeter FM1 that measures a flow rate of a liquid such as the dialysate flowing in the dialysate supply passage 54, the replenisher passage 27B to which the above-described replenisher passage 27A is connected, the blood return passage 23B to which the above-described blood return passage 23A is connected, a third bypass passage 61 provided between the dialysate supply passage 54 and the above-described dialysate collection passage 55, and an eleventh on-off valve V11 that is controlled by the control device 5 are provided.

The above-described replenisher passage 27B is provided to be branched from the dialysate supply passage 54 at a position at a downstream side of the above-described supply side pressure sensor S4, and the replenisher passage 27B is provided with second flow rate restriction device MV2 and a twelfth on-off valve V12 that are controlled by the control device 5.

Further, at an end portion of the replenisher passage 27B, the second connection port P2 disposed on the front surface of the body portion 6 in FIG. 1 is provided, and the connector of the above-described replenisher passage 27A is connected thereto as described above.

When replenishment is performed during dialysis treatment, replenishment to the patient is performed by supplying the dialysate of the dialysate supply passage 54 to the arterial side passage 21 or the venous side passage 22 of the blood circuit 3 via the replenisher passage 27B and the replenisher passage 27A.

At this time, the above-described control device 5 adjusts opening degrees of the above-described first flow rate restriction device MV1 and second flow rate restriction device MV2, and thereby a required amount of dialysate is supplied to the blood circuit 3. Note that control like this is described in Japanese Laid-Open Patent Application No. 2021-062067, for example, and has been conventionally well-known.

The above-described blood return passage 23B is provided by being branched from the dialysate supply passage 54 at the position at a downstream side of the above-described replenisher passage 27B, and is provided with a thirteenth on-off valve V13 that is controlled by the control device 5.

Further, the above-described first connection port P1 provided on the front surface of the body portion 6 in FIG. 1 is provided at an end portion of the blood return passage 23B, and the above-described blood return passage 23 provided by being branched from the arterial side passage 21 of the blood circuit 3 is connected thereto.

When a blood return operation of returning blood in the blood circuit 3 to a patient is performed after dialysis treatment, a fresh dialysate is caused to flow into the blood circuit 3 via the blood return passage 23 from the dialysate supply passage 54 by opening the above-described thirteenth on-off valve V13, and the blood in the blood circuit 3 is pushed back to the patient by the dialysate.

The above-described third bypass passage 61 is branched at a downstream side of the above-described blood return passage 23B to be connected to the above-described dialysate collection passage 55, and includes a fourteenth on-off valve V14 and a fifteenth on-off valve V15 that are controlled by the control device 5.

Between the fourteenth on-off valve V14 and the fifteenth on-off valve in the above-described third bypass passage 61, the above-described connection passage 24B is branched and provided, and the connection passage 24B is provided with a sixteenth on-off valve V16 that is controlled by the control device 5.

At an end portion of the above-described connection passage 24B, the above-described third connection port P3 provided on the front surface of the body portion 6 in FIG. 1 is provided, and the above-describe first priming pipe 24A connected to the arterial side passage 21 of the above-described blood circuit 3 is connected thereto.

The above-described dialysate collection passage 55 has an end portion at an upstream side connected to the above-described dialyzer 2, is connected to an upper side in FIG. 1, and includes, in order from a dialyzer 2 side, a seventeenth on-off valve V17 that is controlled by the control device 5, a collection side pressure sensor S4 that measures a pressure of the dialysate of the dialysate collection passage 55, a gas removal tank 62 that removes air bubbles in the dialysate, the above-described first bypass passage 57 communicating with the above-described liquid supply passage 53, a dialysate pump 63 as liquid delivery device that delivers the dialysate, and a water removal passage 64 for removing water during treatment.

Further, an end portion at a downstream side of the dialysate collection passage 55 is branched in two directions to be connected to the collection chambers 51b and 52b of the above-described first and second dialysate chambers 51 and 52 respectively, and the branched portions are provided with collection valves V18 and V19.

The above-described gas removal tank 62 is provided with a gas discharge passage 65 that has the other end connected to the above-described liquid discharge passage 56, and the gas discharge passage 65 is provided with a twentieth on-off valve V20 that is controlled by the control device 5.

The above-described gas removal tank 62 is conventionally well-known, and when a used dialysate flows in during dialysis treatment, the gas removal tank 62 separates gas contained in the used dialysate, and discharges the gas from the above-described liquid discharge passage 56 via the above-described gas discharge passage 65.

The above-described first bypass passage 57 is connected to between the above-described gas removal tank 62 and a dialysate pump 63, and is used to replenish the dialysate collection passage 55 with clean water of the liquid supply passage 53 during priming operation.

The above-described water removal passage 64 is connected to a downstream side of the dialysate pump 63, and the water removal passage 64 is provided with a water removal pump 66. A second liquid supply passage 67 is provided between the above-described water removal pump 66 and the undiluted dialysate supply device 59, and the second liquid supply passage is provided with a twenty-first on-off valve V21 that is controlled by the control device 5.

By operating the above-described water removal pump 66 during dialysis treatment, a differential pressure is generated between the dialysate container 2b and the blood chamber 2a in the dialyzer 2, and thereby water in the blood is moved to a dialysate circuit 4 side via the membrane of the hollow fibers and removed.

Note that in the present invention, it is also possible to use the above-described water removal pump 66 as liquid delivery device. In this case, supply of clean water is received via the second liquid supply passage 67, and after the clean water flows through the above-described water removal passage 64, the clean water flows through the above-described dialysate collection passage 55 to flow into the collection chambers 51b and 52b of the first and second dialysate chambers 51 and 52.

Then, since a capacity of the collection chamber 51b gradually increases, capacities of the supply chambers 51a and 52a decrease accordingly, and the dialysate can be discharged to the dialysate supply passage 54 from the supply chambers 51a and 52a.

In the above-described liquid discharge passage 56, an upstream portion is branched in two directions to be connected to the collection chambers 51b and 52b of the above-described first and second dialysate chambers 51 and 52, and liquid discharge valves V22 and V23 are provided in the branched portions.

Further, from the above-described branched portions toward the downstream side, the liquid discharge passage 56 is provided with the above-described water removal passage 64 communicating with the above-described dialysate collection passage 55, the above-described gas discharge passage 65 communicating with the gas removal tank 62 of the dialysate collection passage 55, a twenty-fourth on-off valve V24 that is controlled by the control device 5, and the above-described second bypass passage 60 connected to the above-described first bypass passage 57.

Hereinafter, by using the dialysis apparatus 1 having the above-described configuration, a method for determining whether the above-described replenisher passage 27A is connected correspondingly to post-replenishment or pre-replenishment simultaneously with priming will be described.

Note that the priming action described below shows a part of the entire priming action, and the operation described below is only an example, and it is also possible to perform priming and determination of connection of the replenisher passage 27A even if other operations are performed by using the above-described configuration.

Further, in each of FIG. 2 and the subsequent drawings, portions through which the dialysate (or clean water) flows are indicated by a thick line. However, a flow of the dialysate in the dialysate circuit 4 will be described by omitting the thick line for a flow of the dialysate accompanying a normal operation like the operation at the time of dialysis treatment, and indicating a flow of the liquid that occurs with the priming operation by the thick line.

Furthermore, unless otherwise specified, among the respective valves and clamps in the drawings, black ones indicate a closed state, and white ones indicate an open state.

First, by using FIG. 2 and FIG. 3, an operation when the replenisher passage 27A is connected correspondingly to the post-replenishment will be described. At this time, the above-described replenisher passage 27A is connected to the above-described post-replenishment connector C2 of the venous side passage 22 of the blood circuit 3.

First, as shown in FIG. 2, the dialyzer 2 and the blood circuit 3 are fitted to the dialysis apparatus 1. Note that the arterial side passage 21 is not fitted to the blood pump 8, and a flow of the liquid in the arterial side passage 21 is not inhibited.

The blood return passage 23A of the above-described blood circuit 3 is connected to the blood return passage 23B of the dialysate circuit 4 via the first connection port P1. Further, the first priming pipe 24A is connected to the connector 21a of the arterial side passage 21 of the blood circuit 3, and the first priming pipe 24A is connected to the third connection port P3 of the connection passage 24B composing the above-described dialysate circuit 4.

Further, the second priming pipe 25 that is branched from the above-described blood return passage 23B is connected to the connector 22a of the venous side passage 22 of the above-described blood circuit 3, and the manual clamp 26 of the second priming pipe 25 is in an opened state.

From the state where the replenisher passage 27A is connected to the venous side passage 22 corresponding to the post-replenishment like this, a healthcare worker operates the touch panel 5a to instruct start of the priming operation.

In the following explanation, a feeding action of the dialysate in the dialysate circuit 4 will be omitted, and the state in FIG. 2 is a state in which the dialysate is already prepared in the supply chamber 51a of the first dialysate chamber 51, and the supply valve V9 and the collection valve V18 of the collection chamber 51b are opened.

In the above-described liquid supply passage 53, the water supply valve V4 is opened, the liquid supply valve V5 to the supply chamber 51a of the above-described first dialysate chamber 51 is closed, and the seventh on-off valve V7 of the first bypass passage 57 provided between the liquid supply passage 53 and the dialysate collection passage 55 is opened.

Accordingly, when the dialysate pump 63 of the above-described dialysate collection passage 55 performs liquid delivery, the clean water in the above-described liquid supply passage 53 flows through the above-described first bypass passage 57, and thereafter flows into the collection chamber 51b of the above-described first dialysate chamber 51.

Then, since the capacity of the above-described collection chamber 51b gradually increases, and the capacity of the supply chamber 51a decreases accordingly, the dialysate is discharged from the supply chamber 51a to the dialysate supply passage 54.

The discharged dialysate flows through the dialysate supply passage 54, but since the eleventh on-off valve V11 of the dialysate supply passage 54, the thirteenth on-off valve V13 of the blood return passage 23B, and the fourteenth on-off valve V14 of the third bypass passage 61 are closed at this time, the dialysate flows to the replenisher passage 27B from the above-described dialysate supply passage 54.

Further, the replenisher passage 27A is connected to the above-described replenisher passage 27B, and the replenisher passage 27A is connected to the post-replenishment connector C2 of the venous side passage 22 to correspond to the post-replenishment.

Accordingly, the dialysate that flows through the replenisher passage 27B flows into the venous side passage 22 via the above-described post-replenishment connector C2.

In the blood circuit 3, the arterial side clamp V1 of the above-described arterial side passage 21 is opened, and the venous side clamp V2 of the venous side passage 22 is closed.

Accordingly, the dialysate flowing into the venous side passage 22 flows toward the upstream side from the post-replenishment connector C2, but this is a flow in an opposite direction from the flow at the time of dialysis treatment, and thereafter, the dialysate passes through an inside of the dialyzer 2, and thereafter, flows through the above-described arterial side passage 21.

Accordingly, an inside of the above-described arterial side passage 21 is filled with the dialysate, and priming of the arterial side passage 21 can be completed.

The dialysate that flows through the above-described arterial side passage 21 thereafter flows through the above-described first priming pipe 24A, and thereafter flows into the connection passage 24B provided in the above-described dialysate circuit 4.

In the dialysate circuit 4, the seventeenth on-off valve V17 of the dialysate collection passage 55 is closed, the fifteenth on-off valve V15 of the above-described third bypass passage 61 is opened, and the twentieth on-off valve V20 of the gas discharge passage 65 connected to the gas removal tank 62 is opened.

Accordingly, the dialysate flows into the third bypass passage 61 via the above-described connection passage 24B from the above-described first priming pipe 24A, after which, the dialysate flows through the above-described dialysate collection passage 55 to flow into the above-described gas removal tank 62, then flows through the above-described gas discharge passage 65, and thereafter is discharged from the above-described liquid discharge passage 56.

During the above-described operation, the control device 5 measures the pressure of the flowing dialysate by using the supply side pressure sensor S3 provided at the above-described dialysate supply passage 54, and the collection side pressure sensor S4 provided at the above-described dialysate collection passage 55.

Since in the state of FIG. 2, the replenisher passage 27A is connected to the venous side passage 22 correspondingly to the post-replenishment, and the dialysate flows in the opposite direction to a direction at the time of dialysis treatment, as described above, the dialysate is discharged from the above-described arterial side passage 21 after passing through the dialyzer 2 from the venous side passage 22.

When the dialysate passes through the dialyzer 2, a pressure loss occurs when the dialysate passes through the hollow fibers provided in the dialyzer 2, so that the pressure measured by the collection side pressure sensor S4 positioned at the downstream side from the dialyzer 2 becomes lower with respect to the pressure measured by the supply side pressure sensor S3 positioned at the upstream side from the dialyzer 2.

Further, since the venous side clamp V2 in the blood circuit 3 is closed, the control device 5 recognizes that the dialysate flows through the blood circuit 3 in the opposite direction to the direction at the time of dialysis treatment.

When a differential pressure Δ1 between the pressure measured by the above-described supply side pressure sensor S3 and the pressure measured by the collection side pressure sensor S4 exceeds a first threshold that will be described below, in this state, that is, the state where the dialysate flows in the opposite direction, the control device 5 determines that the dialysate flows through the dialyzer 2, and determines that the above-described replenisher passage 27A is connected to the venous side passage 22, that is, correspondingly to the post-replenishment.

When priming of the arterial side passage 21 shown in FIG. 2 is completed, the control device 5 subsequently starts a priming action of the venous side passage 22 as shown in FIG. 3.

The control device 5 opens the venous side clamp V2 of the above-described venous side passage 22, fits the arterial side passage 21 to the above-described blood pump 8, and closes the arterial side passage 21 by the blood pump 8. The manual clamp 26 of the second priming pipe 25 connected to the above-described venous side passage 22 is opened.

When in this state, the dialysate is caused to flow into the blood circuit 3 from the above-described replenisher passage 27A continuously from the state in FIG. 2, the dialysate flows in from the post-replenishment connector C2 provided at the venous side passage 22, and flows in a same forward direction as at the time of dialysis treatment.

As the dialysate flows through the venous side passage 22, the portion at the downstream side from the post-replenishment connector C2 is filled with the dialysate, and since priming of the portion at the upstream side from the post-replenishment connector C2 is completed in the operation of FIG. 2, priming of the venous side passage 22 is completed.

The dialysate flowing through the venous side passage 22 thereafter flows through the second priming pipe 25, thereafter passes through a part of the above-described blood return passage 23B to temporarily flow into the arterial side passage 21, after which, the dialysate flows through the above-described first priming pipe 24A, passes through the above-described dialysate collection passage 55 and thereafter is discharged from the liquid discharge passage 56.

In other words, it is deemed that in a state of FIG. 3, the above-described venous side passage 22 also communicates with the dialysate collection passage 55 via the first priming pipe 24A and the second priming pipe 25 as the connection passages, and the connection passage 24B.

Since in the operation shown in FIG. 3, the dialysate flows through only the venous side passage 22 to be discharged to the dialysate collection passage 55, the dialysate does not pass through the dialyzer 2, and therefore, a pressure loss due to the dialyzer 2 does not occur unlike the priming of the arterial side passage 21.

On the other hand, the dialysate passes through the drip chamber 9 provided at the venous side passage 22, and the check valve B provided at the blood return passage 23A in which the dialysate flows via the above-described second priming pipe 25, and a pressure loss caused by the dialysate passing through them occurs. However, the pressure loss in this case is lower than the pressure loss caused by passing through the dialyzer 2.

When a differential pressure Δ2 does not exceed a second threshold described below in the state where the dialysate flows in the forward direction, the control device 5 determines that the dialysate does not flow through the dialyzer 2, and the above-described replenisher passage 27A is connected to the venous side passage 22, that is, correspondingly to the post-replenishment.

Here, the case where the replenisher passage 27A is connected to the pre-replenishment connector C1 of the arterial side passage 21 due to a connection error as shown in FIG. 4 and FIG. 5 although an instruction to perform post-replenishment is given will be described.

First, in FIG. 4 corresponding to FIG. 2, the dialysate flows in from the pre-replenishment connector C1 provided in the arterial side passage 21, from the replenisher passage 27A, and thereafter, flows in the opposite direction to the direction at the time of dialysis treatment.

Since the dialysate flows in from the arterial side passage 21, and is discharged from the above-described first priming pipe 24A without passing through the dialyzer 2, a pressure loss caused by passing through the dialyzer 2 as described above does not occur.

Accordingly, a differential pressure Δ3 between the pressure measured by the supply side pressure sensor S3 and the pressure measured by the collection side pressure sensor S4 becomes smaller than in the case of connection corresponding to the post-replenishment, and does not exceed the above-described first threshold.

In other words, when the above-described differential pressure Δ3 does not exceed the first threshold in the state where the dialysate flows in the opposite direction, the control device 5 determines that the replenisher passage 27A is connected to the arterial side passage 21, that is, correspondingly to pre-replenishment, and displays a warning on the above-described touch panel 5a or the like to the effect that there is an error in the connection.

Note that connection check like this can also be performed when the dialysate flows in the same forward direction as at the time of dialysis treatment as shown in FIG. 5.

That is to say, when the dialysate flows in from the pre-replenishment connector C1 provided at the arterial side passage 21, and flows in the same forward direction as at the time of dialysis treatment, a pressure loss is generated by the dialysate passing through the dialyzer 2, thereafter passing through the drip chamber 9 of the venous side passage 22, and the second priming pipe 25, and thereafter passing through the check valve B of the blood return passage 23A, so that a differential pressure Δ4 between the pressure measured by the supply side pressure sensor S3 and the pressure measured by the collection side pressure sensor S4 exceeds the above-described second threshold.

Accordingly, when the above-described differential pressure Δ4 exceeds the second threshold in the state where the dialysate flows in the forward direction, the control device 5 can determine that the replenisher passage 27A is connected to the arterial side passage 21, that is, correspondingly to the pre-replenishment.

Next, by using FIG. 4 and FIG. 5, an operation when the replenisher passage 27A is connected correspondingly to the pre-replenishment will be described. At this time, with respect to the states in FIG. 2 and FIG. 3, the replenisher passage 27A is connected to the above-described pre-replenishment connector C1 of the arterial side passage 21.

When start of the priming operation is instructed from this state, the dialysate discharged from the supply chamber 51a of the first dialysate chamber 51 of the dialysate circuit 4 flows to the replenisher passage 27A from the above-described dialysate supply passage 54.

In the state of FIG. 4, in the blood circuit 3, the arterial side clamp V1 of the above-described arterial side passage 21 is opened, and the venous side clamp V2 of the venous side passage 22 is closed.

Accordingly, the dialysate flowing into the arterial side passage 21 via the pre-replenishment connector C1 from the replenisher passage 27A flows in the opposite direction to the direction at the time of dialysis treatment, and flows through the above-described first priming pipe 24A, thereafter flows through the above-described dialysate collection passage 55, flows through the gas discharge passage 65 from the above-described gas removal tank 62, and thereafter is discharged from the above-described liquid discharge passage 56.

By causing the dialysate to flow in the opposite direction to the direction of the normal dialysis treatment in this way, it is possible to fill the portion at the upstream side from the pre-replenishment connector C1 of the above-described arterial side passage 21 in the blood circuit 3 with the dialysate.

On the other hand, during the above-described operation, the control device 5 measures the pressure of the flowing dialysate by using the supply side pressure sensor S3 provided at the above-described dialysate supply passage 54, and the collection side pressure sensor S4 provided at the above-described dialysate collection passage 55.

Here, as described above, in the state of FIG. 4, the replenisher passage 27A is provided at the arterial side passage 21 corresponding to the pre-replenishment, and the dialysate flows in the opposite direction to the direction at the time of dialysis treatment, and therefore, the dialysate is discharged from the above-describe arterial side passage 21 without passing through the dialyzer 2 after flowing in from the arterial side passage 21.

Accordingly, the pressure loss when the above-describe dialysate passes through the dialyzer 2 does not occur, and the pressure loss by the drip chamber 9 and the check valve B does not occur either, as in FIG. 3.

From the above, the replenisher passage 27A is connected correspondingly to the pre-replenishment as in FIG. 4, and when the dialysate flows in the opposite direction, a value of the differential pressure Δ3 between the pressure measured by the supply side pressure sensor S3 positioned at the upstream side with respect to the dialyzer 2, and the pressure measured by the collection side pressure sensor S4 positioned at the downstream side with respect to the dialyzer 2 becomes small.

The control device 5 determines that the above-described replenisher passage 27A is connected to the arterial side passage 21, that is, correspondingly to the pre-replenishment, when the differential pressure Δ3 between the pressure measured by the supply side pressure sensor S3 and the pressure measured by the collection side pressure sensor S4 does not exceed the first threshold used in the state of FIG. 2 described above.

When the dialysate is discharged from the replenisher passage 27A connected to the arterial side passage 21 correspondingly to the pre-replenishment in this way, and priming of the arterial side passage 21 is completed, the dialysate is subsequently caused to flow in the same forward direction as at the time of the dialysis treatment as shown in FIG. 5.

In this case, the control device 5 opens the venous side clamp V2 of the above-described venous side passage 22, and fits the arterial side passage 21 to the above-described blood pump 8, and closes the arterial side passage 21 by the blood pump 8. Further, the manual clamp 26 of the second priming pipe 25 connected to the above-described venous side passage 22 is opened.

When the dialysate is caused to flow into the blood circuit 3 via the above-described replenisher passage 27A in this state, the dialysate that flows in from the pre-replenishment connector C1 of the arterial side passage 21 flows through the arterial side passage 21 in the same forward direction as at the time of dialysis treatment.

The dialysate flows through the above-described venous side passage 22 after passing through the above-described dialyzer 2, and thereafter, passes through the second priming pipe 25 and a part of the arterial side passage 21, flows through the above-described first priming pipe 24A, and thereafter is discharged from the dialysate collection passage 55 via the liquid discharge passage 56.

In the operation shown in FIG. 5, pressure losses occur due to the dialysate passing through the dialyzer 2, and passing through the drip chamber 9 of the venous side passage 22 and the check valve of the blood return passage 23A.

Accordingly, when the differential pressure Δ4 between the pressure measured by the supply side pressure sensor S3 and the pressure measured by the collection side pressure sensor S4 exceeds the above-described second threshold used in FIG. 3 described above in the state where the dialysate flows in the forward direction, the control device 5 judges that the dialysate flows through the dialyzer 2, and determines that the above-described replenisher passage 27A is connected to the arterial side passage 21, that is, correspondingly to the pre-replenishment.

Here, an operation when the replenisher passage 27A is connected to the post-replenishment connector C2 of the venous side passage 22 due to a connection error will be described.

When the dialysate is caused to flow in the opposite direction in the state where the replenisher passage 27A is connected to the post-replenishment connector C2, a pressure loss occurs by passing through the dialyzer 2 as shown in FIG. 2, and the differential pressure Δ1 is measured as described with FIG. 2.

Since the above-described differential pressure Δ1 exceeds the above-described first threshold, the control device 5 can recognize that there is an error in the connection.

Similarly, when the dialysate is caused to flow in the forward direction in the state where the replenisher passage 27A is connected to the post-replenishment connector C2, the dialysate flows in from the venous side passage 22 as shown in FIG 3 and is discharged without passing through the dialyzer 2, and therefore the above-described differential pressure Δ2 is measured as described in FIG. 3.

Since the above-described differential pressure Δ2 does not exceed the above-described second threshold, the control device 5 can recognize that there is an error in the connection.

Hereinafter, a method (not being part of the present invention) for setting the above-described first threshold and second threshold will be described.

First, concerning the first threshold, the replenisher passage 27A is connected to the post-replenishment connector C2 of the venous side passage 22 correspondingly to the post-replenishment, and the dialysate is caused to flow in the opposite direction, as shown in FIG. 2.

Subsequently, the differential pressure Δ1 between the pressure measured by the supply side pressure sensor S3 and the pressure measured by the collection side pressure sensor S4 is measured.

Subsequently, as shown in FIG. 4, the replenisher passage 27A is connected to the pre-replenishment connector C1 of the arterial side passage 21 correspondingly to the pre-replenishment, and the dialysate is caused to flow in the opposite direction.

Then, the differential pressure Δ3 between the pressure measured by the supply side pressure sensor S3, and the pressure measured by the collection side pressure sensor S4 is measured.

A value between the differential pressure Δ1 and the differential pressure Δ3 that are measured in this way can be made the first threshold, and, for example, an intermediate value between the differential pressure Δ1 and the differential pressure Δ3 can be made the first threshold.

Similarly, concerning the second threshold, the replenisher passage 27A is connected to the post-replenishment connector C2 of the venous side passage 22 correspondingly to the post-replenishment, and the dialysate is caused to flow in the forward direction, as shown in FIG. 3.

Subsequently, the differential pressure Δ2 between the pressure measured by the supply side pressure sensor S3 and the pressure measured by the collection side pressure sensor S4 is measured.

Subsequently, as shown in FIG. 5, the replenisher passage 27A is connected to the pre-replenishment connector C1 of the arterial side passage 21 correspondingly to the pre-replenishment, and the dialysate is caused to flow in the forward direction.

Then, the differential pressure Δ4 between the pressure measured by the supply side pressure sensor S3 and the pressure measured by the collection side pressure sensor S4 is measured.

A value between the differential pressure Δ2 and the differential pressure Δ4 that are measured in this way can be made the second threshold, and, for example, an intermediate value between the differential pressure Δ2 and the differential pressure Δ4 can be made the second threshold.

Here, when the dialysate is caused to flow in the opposite direction as shown in FIG. 2 and FIG. 4, in the connection corresponding to the post-replenishment in FIG. 2, the dialysate passes through the dialyzer 2, and when the differential pressure Δ1 at this time is actually measured, the differential pressure Δ1 = 45 kPa.

In contrast, in the connection corresponding to the pre-replenishment in FIG. 3, the dialysate does not pass through the dialyzer 2, and when the differential pressure Δ3 at this time is measured, the differential pressure Δ3 = 40 kPa.

Accordingly, a difference between the differential pressure Δ1 and the differential pressure Δ3 is 5 kPa, and an intermediate value 42.5 kPa that is an intermediate value of them can be set as the first threshold.

However, the difference between the differential pressure Δ1 and the differential pressure Δ3 is only 5 kPa, and therefore, depending on measurement situations (state of layout of the blood circuit or the like) of the supply side pressure sensor S3 and the collection side pressure sensor S4, the differential pressure Δ1 may be detected as only 42 kPa in the state of FIG. 2.

Because in that case, the differential pressure Δ1 is smaller than the first threshold, the control device 5 judges that the replenisher passage 27A is not connected correspondingly to the post-replenishment, and false determination occurs.

This also applies to the case in which the dialysate is caused to flow in the forward direction, and when actually measuring the differential pressure based on the pipes in FIG. 3, the differential pressure Δ2 = 60 kPa, and according to the differential pressure Δ4 = 65 kPa that is measured based on the pipes of FIG. 5, a difference between the differential pressure Δ2 and the differential pressure Δ4 is only 5 kPa.

Thus, in order to perform connection check of the replenisher passage 27A more accurately, it is possible to perform connection check using the third threshold that will be described below.

In the state where the replenisher passage 27A is connected correspondingly to the post-replenishment shown in FIG. 2 and FIG. 3, a difference between the differential pressure Δ3 = 45 kPa in the case of causing the dialysate to flow in the opposite direction shown in FIG. 2, and the differential pressure Δ4 = 60 kPa in the case of causing the dialysate to flow in the forward direction is Δpost = 15 kPa (absolute value).

In contrast, the difference between the differential pressure Δ1 = 40 kPa in the case of causing the dialysate to flow in the opposite direction shown in FIG. 4, and the differential pressure Δ2 = 65 kPa in the case of causing the dialysate to flow in the forward direction is Δpre = 25 kPa (absolute value) in the state where the replenisher passage 27A is connected correspondingly to the pre-replenishment shown in FIG. 4 and FIG. 5.

A difference between Δpost = 15 kPa in the case of corresponding to the above-described post-replenishment and Δpre = 25 kPa in the case of corresponding to the pre-replenishment is 10 kPa, which is larger than the difference between the differential pressure Δ1 and the differential pressure Δ3 described above, and the difference between the differential pressure Δ2 and the differential pressure Δ4.

Then, 20 kPa that is the intermediate value between Δpost and Δpre can be used as the third threshold. At this time, Δpost corresponding to the post-replenishment is lower than the above-described third threshold, and Δpre corresponding to the pre-replenishment is higher than the above-described third threshold.

In the third threshold, the difference between the above-described Δpost and Δpre is 10 kPa, and therefore, an allowable range of a measurement error becomes larger than the aforementioned first threshold and second threshold, and it becomes possible to perform connection check more reliably.

Hereinafter, a method (not being part of the invention) for checking connection actually using the third threshold will be described.

First, the case in which the replenisher passage 27A is connected to the post-replenishment connector C2 correspondingly to the post-replenishment shown in FIG. 2 and FIG. 3 will be described.

In the state where the dialysate is caused to flow in the opposite direction as shown in FIG. 2, the differential pressure Δ1 is measured by the above-described supply side pressure sensor S3 and collection side pressure sensor S4, and further, the differential pressure Δ2 is measured in the state where the dialysate is caused to flow in the forward direction as shown in FIG. 3.

Subsequently, after the differential pressure Δ2 is measured, the control device 5 calculates the difference Δpost between the above-described differential pressure Δ1 and differential pressure Δ2, compares the difference Δpost with the third threshold, and when the Δpost is lower than the third threshold, the control device 5 can determine that the replenisher passage 27A is connected correspondingly to the post-replenishment.

In the case in which the replenisher passage 27A is connected to the pre-replenishment connector C1 correspondingly to the pre-replenishment shown in FIG. 4 and FIG. 5 on the contrary, determination can also be performed similarly.

In the state where the dialysate is caused to flow in the opposite direction as shown in FIG. 4, the differential pressure Δ3 is measured by the above-described supply side pressure sensor S3 and collection side pressure sensor S4, and further, in the state where the dialysate is caused to flow in the forward direction as shown in FIG. 5, the differential pressure Δ4 is measured.

After the differential pressure Δ4 is measured, the control device 5 calculates the difference Δpre between the above-described differential pressure Δ3 and differential pressure Δ4, compares the difference Δpre with the third threshold, and when the Δpre exceeds the third threshold, the control device 5 can determine that the replenisher passage 27A is connected correspondingly to the pre-replenishment.

Note that in the above-described embodiment, the so-called personal dialysis apparatus 1 that prepares the dialysate by adding the dialysate undiluted solution from the dialysate undiluted solution supply device to the clean water supplied by the liquid supply device and supplying them is described, but a so-called console type dialysis apparatus 1 that supplies the dialysate prepared in advance by the above-described liquid supply device may be used.

Note that in the above-described embodiment, in order to determine the connection check of the replenisher passage 27A in parallel with the priming action, it is necessary to perform operation shown in FIG. 2 and FIG. 3 (or FIG. 4 and FIG. 5) in order to fill both the arterial side passage 21 and the venous side passage 22 with the dialysate.

However, if the above-described determination operation is performed after the priming action is completed, it is possible to perform determination by the operation of either one of FIG. 2 or FIG. 3 (or either one of FIG. 4 or FIG. 5).

In other words, if a required threshold that is set in advance is used with respect to the differential pressure Δ1 of the pressures measured by the supply side pressure sensor S3 and the collection side pressure sensor S4 in the state where the dialysate is caused to flow in the opposite direction shown in FIG. 2, for example, it is possible to perform determination by only the operation shown in FIG. 2.

### Reference Signs List

| | | | |
|---|---|---|---|
| 1 | dialysis apparatus | 2 | dialyzer |
| 3 | blood circuit | 4 | dialysate circuit |
| 21 | arterial side passage | 22 | venous side passage |
| 24A | first priming pipe (connection passage) | | |
| 24B | connection passage | | |
| 25 | second priming pipe (connection passage) | | |
| 27A, B | replenisher passage | | |
| 54 | dialysate supply passage | | |
| 55 | dialysate collection passage | | |
| 65 | dialysate pump (liquid delivery device) | | |
| C1 | pre-replenishment connector | | |
| C2 | post-replenishment connector | | |

## Claims

1. A dialysis apparatus (1) comprising a dialyzer (2) that performs hemodialysis, a dialysate supply passage (54) that supplies a fresh dialysate to the dialyzer (2), a dialysate collection passage (55) that collects a used dialysate that passes through the dialyzer (2), a blood circuit (3) comprising an arterial side passage (21) that supplies blood to the dialyzer (2) and a venous side passage (22) that discharges blood from the dialyzer (2), a replenisher passage (27A, 27B) provided between the dialysate supply passage (54) and the blood circuit (3), and liquid delivery device (65) that delivers the dialysate of the dialysate supply passage (54) to the blood circuit (3) via the replenisher passage (27A, 27B),
the replenisher passage (27A, 27B) being provided to be selectively connectable to the arterial side passage (21) or the venous side passage (22) of the blood circuit (3), the dialysis apparatus (1) being **characterized by** comprising
a supply side pressure sensor (S3) that measures a pressure of a liquid flowing through the dialysate supply passage (54), a collection side pressure sensor (S4) that measures a pressure of a liquid flowing through the dialysate collection passage (55), and a connection passage (24A, 24B, 25) in which one end communicates with the arterial side passage (21) or the venous side passage (22) of the blood circuit (3), and the other end communicates with the dialysate collection passage (55), and **characterized in that**
determination device (5) is further provided, the determination device (5) determining whether the replenisher passage (27A, 27B) is connected to the arterial side passage (21), or the venous side passage (22) based on a differential pressure between a pressure measured by the supply side pressure sensor (S3) and a pressure measured by the collection side pressure sensor (S4), in a state where the liquid delivery device (65) causes a liquid to flow into the blood circuit (3) from the replenisher passage (27A, 27B), and the liquid flows through the dialysate collection passage (55) via the connection passage (24A, 24B, 25) from the arterial side passage (21) or the venous side passage (22).

2. The dialysis apparatus (1) according to claim 1,
**characterized in that**
the determination device (5) calculates a differential pressure between a pressure measured by the supply side pressure sensor (S3) and a pressure measured by the collection side pressure sensor (S4) in a state where the liquid which the liquid delivery device (65) delivers to the blood circuit (3) from the replenisher passage (27A, 27B) is caused to flow out to the dialysate collection passage (55) via the connection passage (24A, 24B, 25) from an end portion of the arterial side passage (21), and
when the differential pressure is smaller than a first threshold, the determination device (5) determines that the replenisher passage (27A, 27B) is connected to the arterial side passage (21), and when the differential pressure is larger than the first threshold, the determination device (5) determines that the replenisher passage (27A, 27B) is connected to the venous side passage (22).

3. The dialysis apparatus (1) according to claim 1,
**characterized in that**
the determination device (5) calculates a differential pressure between a pressure measured by the supply side pressure sensor (S3) and a pressure measured by the collection side pressure sensor (S4), in a state where the liquid which the liquid delivery device (65) delivers to the blood circuit (3) from the replenisher passage (27A, 27B) is caused to flow out to the dialysate collection passage (55) via the connection passage (24A, 24B, 25) from an end portion of the venous side passage (22), and
when the differential pressure is smaller than a second threshold, the determination device (5) determines that the replenisher passage (27A, 27B) is connected to the venous side passage (22), and when the differential pressure is larger than the second threshold, the determination device (5) determines that the replenisher passage (27A, 27B) is connected to the arterial side passage (21).

4. A method for checking connection of a replenisher passage (27A, 27B) before performing dialysis treatment and not including dialysis treatment, including a dialyzer (2) that performs hemodialysis, a dialysate supply passage (54) that supplies a fresh dialysate to the dialyzer (2), a dialysate collection passage (55) that collects a used dialysate that passes through the dialyzer (2), a blood circuit (3) comprising an arterial side passage (21) that supplies blood to the dialyzer (2) and a venous side passage (22) that discharges blood from the dialyzer (2), a replenisher passage (27A, 27B) provided between the dialysate supply passage (54) and the blood circuit (3), and liquid delivery device (65) that delivers the dialysate in the dialysate supply passage (54) to the blood circuit (3) via the replenisher passage (27A, 27B), and determining whether the replenisher passage (27A, 27B) is connected to the arterial side passage (21), or the venous side passage (22) in the blood circuit (3), the method being **characterized in that**
a supply side pressure sensor (S3) that measures a pressure in the dialysate supply passage (54), and a collection side pressure sensor (S4) that measures a pressure in the dialysate collection passage (55) are provided,
in a state where either one of the arterial side passage (21) or the venous side passage (22) of the blood circuit (3) is caused to communicate with the dialysate collection passage (55), the liquid delivery device (65) causes a liquid to flow into the blood circuit (3) from the replenisher passage (27A, 27B), and the liquid is caused to flow to the dialysate collection passage (55) from the arterial side passage (21) or the venous side passage (22), and
it is determined whether a connection destination of the replenisher passage (27A, 27B) is the arterial side passage (21), or the venous side passage (22) based on a differential pressure between a pressure measured by the supply side pressure sensor (S3) and a pressure measured by the collection side pressure sensor (S4).

5. The method for checking connection of a replenisher passage (27A, 27B) according to claim 4, **characterized by** comprising:
calculating a differential pressure between a pressure measured by the supply side pressure sensor (S3) and a pressure measured by the collection side pressure sensor (S4), in a state where the liquid delivered to the blood circuit (3) from the replenisher passage (27A, 27B) by the liquid delivery device (65) is caused to flow out to the dialysate collection passage (55) via the connection passage (24A, 24B, 25) from an end portion of the arterial side passage (21), and
determining that the replenisher passage (27A, 27B) is connected to the arterial side passage (21) when the differential pressure is smaller than a first threshold, and determining that the replenisher passage (27A, 27B) is connected to the venous side passage (22) when the differential pressure is larger than the first threshold.

6. The method for checking connection of a replenisher passage (27A, 27B) according to claim 4, **characterized by** comprising:
calculating a differential pressure between a pressure measured by the supply side pressure sensor (S3) and a pressure measured by the collection side pressure sensor (S4) in a state where the liquid delivered to the blood circuit (3) from the replenisher passage (27A, 27B) by the liquid delivery device (65) is caused to flow out to the dialysate collection passage (55) via the connection passage (24A, 24B, 25) from an end portion of the venous side passage (22), and
determining that the replenisher passage (27A, 27B) is connected to the venous side passage (22) when the differentia pressure is smaller than a second threshold, and determining that the replenisher passage (27A, 27B) is connected to the arterial side passage (21) when the differential pressure is larger than the second threshold.

## Patentansprüche

1. Dialysevorrichtung (1), die einen Dialysator (2), der eine Hämodialyse ausführt, einen Dialysatversorgungskanal (54), der frisches Dialysat zum Dialysator (2) zuführt, einen Dialysatsammelkanal (55), der verbrauchtes Dialysat sammelt, das den Dialysator (2) passiert, einen Blutkreislauf (3), der einen arterienseitigen Kanal (21), der Blut zum Dialysator (2) zuführt, und einen venenseitigen Kanal (22), der Blut aus dem Dialysator (2) abführt, einen Replenisher-Kanal (27A, 27B), der zwischen dem Dialysatversorgungskanal (54) und dem Blutkreislauf (3) bereitgestellt ist, und eine Flüssigkeitszufuhrvorrichtung (65), die das Dialysat des Dialysatversorgungskanals (54) zum Blutkreislauf (3) über den Replenisher-Kanal (27A, 27B) zuführt,
wobei der Replenisher-Kanal (27A, 27B) so bereitgestellt ist, dass er selektiv mit dem arterienseitigen Kanal (21) oder dem venenseitigen Kanal (22) des Blutkreislaufs (3) verbindbar ist, wobei die Dialysevorrichtung (1) **dadurch gekennzeichnet ist, dass** es Folgendes umfasst:
einen versorgungsseitigen Drucksensor (S3), der den Druck einer Flüssigkeit misst, die durch den Dialysatversorgungskanal (54) strömt, einen sammelseitigen Drucksensor (S4), der den Druck einer Flüssigkeit misst, die durch den Dialysatsammelkanal (55) strömt, und einen Verbindungskanal (24A, 24B, 25), bei dem ein Ende mit dem arterienseitigen Kanal (21) oder dem venenseitigen Kanal (22) des Blutkreislaufs (3) kommuniziert und das andere Ende mit dem Dialysatsammelkanal (55) kommuniziert, und **dadurch gekennzeichnet, dass**
weiters eine Bestimmungsvorrichtung (5) bereitgestellt ist, wobei die Bestimmungsvorrichtung (5) bestimmt, ob der Replenisher-Kanal (27A, 27B) mit dem arterienseitigen Kanal (21) oder dem venenseitigen Kanal (22) verbunden ist, basierend auf einer Druckdifferenz zwischen einem Druck, der vom versorgungsseitigen Drucksensor (S3) gemessen wird, und einem Druck der vom sammelseitigen Drucksensor (S4) gemessen wird, und zwar in einem Zustand, in dem die Flüssigkeitszufuhrvorrichtung (65) bewirkt, dass eine Flüssigkeit vom Replenisher-Kanal (27A, 27B) in den Blutkreislauf (3) strömt und die Flüssigkeit über den Verbindungskanal (24A, 24B, 25) vom arterienseitigen Kanal (21) oder vom venenseitigen Kanal (22) durch den Dialysatsammelkanal (55) strömt.

2. Dialysevorrichtung (1) nach Anspruch 1, **dadurch gekennzeichnet, dass**
die Bestimmungsvorrichtung (5) eine Druckdifferenz zwischen einem Druck, der vom versorgungsseitigen Drucksensor (S3) gemessen wird, und einem Druck, der vom sammelseitigen Drucksensor (S4) gemessen wird, bestimmt, und zwar in einem Zustand, in dem bewirkt wird, dass die Flüssigkeit, welche die Flüssigkeitszufuhrvorrichtung (65) vom Replenisher-Kanal (27A, 27B) zum Blutkreislauf (3) zuführt, über den Verbindungskanal (24A, 24B, 25) von einem Endabschnitt des arterienseitigen Kanals (21) aus dem Dialysatsammelkanal (55) ausströmt, und
wenn die Druckdifferenz kleiner ist als ein erster Schwellwert, die Bestimmungsvorrichtung (5) bestimmt, dass der Replenisher-Kanal (27A, 27B) mit dem arterienseitigen Kanal (21) verbunden ist, und wenn die Druckdifferenz größer ist als die erste Schwelle, die Bestimmungsvorrichtung (5) bestimmt, dass der Replenisher-Kanal (27A, 27B) mit dem venenseitigen Kanal (22) verbunden ist.

3. Dialysevorrichtung (1) nach Anspruch 1, **dadurch gekennzeichnet, dass**
die Bestimmungsvorrichtung (5) eine Druckdifferenz zwischen einem Druck, der vom versorgungsseitigen Drucksensor (S3) gemessen wird, und einem Druck, der vom sammelseitigen Drucksensor (S4) gemessen wird, berechnet, und zwar in einem Zustand, in dem bewirkt, wird, dass die Flüssigkeit, welche die Flüssigkeitszufuhrvorrichtung (65) vom Replenisher-Kanal (27A, 27B) zum Blutkreislauf (3) zuführt, über den Verbindungskanal (24A, 24B, 25) von einem Endabschnitt des venenseitigen Kanals (22) in den Dialysatsammelkanal (55) ausströmt, und
wenn die die Druckdifferenz kleiner ist als ein zweiter Schwellwert, die Bestimmungsvorrichtung (5) bestimmt, dass der Replenisher-Kanal (27A, 27B) mit dem venenseitigen Kanal (22) verbunden ist, und wenn die Druckdifferenz größer als der zweite Schwellwert ist, die Bestimmungsvorrichtung (5) bestimmt, dass der Replenisher-Kanal (27A, 27B) mit dem arterienseitigen Kanal (21) verbunden ist.

4. Verfahren zum Prüfen der Verbindung eines Replenisher-Kanals (27A, 27B) vor der Durchführung einer Dialysebehandlung und nicht die Dialysebehandlung einschließend, umfassend einen Dialysator (2), der eine Hämodialyse ausführt, einen Dialysatversorgungskanal (54), der frisches Dialysat zum Dialysator (2) zuführt, einen Dialysatsammelkanal (55), der verbrauchtes Dialysat sammelt, das den Dialysator (2) passiert, einen Blutkreislauf (3), der einen arterienseitigen Kanal (21), der Blut zum Dialysator (2) zuführt, und einen venenseitigen Kanal (22), der Blut aus dem Dialysator (2) abführt, einen Replenisher-Kanal (27A, 27B), der zwischen dem Dialysatversorgungskanal (54) und dem Blutkreislauf (3) bereitgestellt ist, und eine Flüssigkeitszufuhrvorrichtung (65), die das Dialysat des Dialysatversorgungskanals (54) zum Blutkreislauf (3) über den Replenisher-Kanal (27A, 27B) zuführt, und das Bestimmen, ob der Replenisher-Kanal (27A, 27B) mit dem arterienseitigen Kanal (21) oder dem venenseitigen Kanal (22) des Blutkreislaufs (3) verbunden ist, wobei das Verfahren **dadurch gekennzeichnet, dass** ist, dass
ein versorgungsseitiger Drucksensor (S3), der den Druck im Dialysatversorgungskanal (54) misst, und ein sammelseitiger Drucksensor (S4), der den im Dialysatsammelkanal (55) misst, bereitgestellt sind,
wobei in einem Zustand, in dem bewirkt wird, dass einer aus dem arterienseitigen Kanal (21) oder dem venenseitigen Kanal (22) des Blutkreislaufs (3) mit dem Dialysatsammelkanal (55) kommuniziert, die Flüssigkeitszufuhrvorrichtung (65) bewirkt, dass eine Flüssigkeit vom Replenisher-Kanal (27A, 27B) in den Blutkreislauf (3) strömt, und bewirkt wird, dass die Flüssigkeit vom arterienseitigen Kanal (21) oder vom venenseitigen Kanal (22) in den Dialysatsammelkanal (55) strömt, und
bestimmt wird, ob die Verbindungsdestination des Replenisher-Kanals (27A, 27B) der arterienseitige Kanal (21) oder der venenseitige Kanal (22) ist, basierend auf einer Druckdifferenz zwischen einem Druck, der vom versorgungsseitigen Drucksensor (S3) gemessen wird, und einem Druck, der vom sammelseitigen Drucksensor (S4) gemessen wird.

5. Verfahren zum Prüfen der Verbindung eines Replenisher-Kanals (27A, 27B) nach Anspruch 4, **dadurch gekennzeichnet, dass** es Folgendes umfasst:
Berechnen einer Druckdifferenz zwischen einem Druck, der vom versorgungsseitigen Drucksensor (S3) gemessen wird, und einem Druck, der vom sammelseitigen Drucksensor (S4) gemessen wird, und zwar in einem Zustand, in dem bewirkt wird, dass die Flüssigkeit, die durch die Flüssigkeitszufuhrvorrichtung (65) vom Replenisher-Kanal (27A, 27B) zum Blutkreislauf (3) zugeführt wird, über den Verbindungskanal (24A, 24B, 25) von einem Endabschnitt des arterienseitigen Kanals (21) zu dem Dialysatsammelkanal (55) ausströmt, und
Bestimmen, dass der Replenisher-Kanal (27A, 27B) mit dem arterienseitigen Kanal (21) verbunden ist, wenn die Druckdifferenz kleiner ist als ein erster Schwellwert, und Bestimmen, dass der Replenisher-Kanal (27A, 27B) mit dem venenseitigen Kanal (22) verbunden ist, wenn die Druckdifferenz größer ist als der erste Schwellwert.

6. Verfahren zum Prüfen der Verbindung eines Replenisher-Kanals (27A, 27B) nach Anspruch 4, **dadurch gekennzeichnet, dass** es Folgendes umfasst:
Berechnen der Druckdifferenz zwischen einem Druck, der vom versorgungsseitigen Drucksensor (S3) gemessen wird, und einem Druck, der vom sammelseitigen Drucksensor (S4) gemessen wird, und zwar in einem Zustand, in dem bewirkt wird, dass die Flüssigkeit, die vom Replenisher-Kanal (27A, 27B) durch die Flüssigkeitszufuhrvorrichtung (65) zum Blutkreislauf (3) zugeführt wird, über den Verbindungskanal (24A, 24B, 25) von einem Endabschnitt des venenseitigen Kanals (22) zu dem Dialysatsammelkanal (55) ausströmt, und
Bestimmen, dass der Replenisher-Kanal (27A, 27B) mit dem venenseitigen Kanal (22) verbunden ist, wenn die Druckdifferenz kleiner als ein zweiter Schwellwert ist, und Bestimmen, dass der Replenisher-Kanal (27A, 27B) mit dem arterienseitigen Kanal (21) verbunden ist, wenn die Druckdifferenz größer als der zweite Schwellwert ist.

## Revendications

1. Appareil de dialyse (1) comprenant un dialyseur (2) qui réalise une hémodialyse, un passage d'alimentation en dialysat (54) qui fournit un dialysat frais au dialyseur (2), un passage de collecte de dialysat (55) qui collecte un dialysat usagé qui passe à travers le dialyseur (2), un circuit sanguin (3) comprenant un passage côté artériel (21) qui fournit du sang au dialyseur (2) et un passage côté veineux (22) qui évacue le sang du dialyseur (2), un passage de régénération (27A, 27B) prévu entre le passage d'alimentation en dialysat (54) et le circuit sanguin (3), et le dispositif de distribution de liquide (65) qui distribue le dialysat du passage d'alimentation en dialysat (54) au circuit sanguin (3) via le passage de régénération (27A, 27B),
le passage de régénération (27A, 27B) étant prévu pour pouvoir être raccordé sélectivement au passage côté artériel (21) ou au passage côté veineux (22) du circuit sanguin (3), l'appareil de dialyse (1) étant **caractérisé en ce qu'**il comprend
un capteur de pression côté alimentation (S3) qui mesure une pression d'un liquide s'écoulant à travers le passage d'alimentation en dialysat (54), un capteur de pression côté collecte (S4) qui mesure une pression d'un liquide s'écoulant à travers le passage de collecte de dialysat (55), et un passage de raccordement (24A, 24B, 25) dans lequel une extrémité communique avec le passage côté artériel (21) ou le passage côté veineux (22) du circuit sanguin (3), et l'autre extrémité communique avec le passage de collecte de dialysat (55) et **caractérisé en ce que**
un dispositif de détermination (5) est en outre prévu, le dispositif de détermination (5) déterminant si le passage de régénération (27A, 27B) est raccordé au passage côté artériel (21), ou au passage côté veineux (22) sur la base d'une pression différentielle entre une pression mesurée par le capteur de pression côté alimentation (S3) et une pression mesurée par le capteur de pression côté collecte (S4), dans un état où le dispositif de distribution de liquide (65) amène un liquide à s'écouler dans le circuit sanguin (3) à partir du passage de régénération (27A, 27B), et le liquide s'écoule à travers le passage de collecte de dialysat (55) via le passage de raccordement (24A, 24B, 25) à partir du passage côté artériel (21) ou du passage côté veineux (22).

2. Appareil de dialyse (1) selon la revendication 1, **caractérisé en ce que**
le dispositif de détermination (5) calcule une pression différentielle entre une pression mesurée par le capteur de pression côté alimentation (S3) et une pression mesurée par le capteur de pression côté collecte (S4) dans un état où le liquide que distribue le dispositif de distribution de liquide (65) au circuit sanguin (3) à partir du passage de régénération (27A, 27B) est amené à s'écouler vers le passage de collecte de dialysat (55) via le passage de raccordement (24A, 24B, 25) à partir d'une partie d'extrémité du passage côté artériel (21), et
lorsque la pression différentielle est inférieure à un premier seuil, le dispositif de détermination (5) détermine que le passage de régénération (27A, 27B) est raccordé au passage côté artériel (21), et lorsque la pression différentielle est supérieure au premier seuil, le dispositif de détermination (5) détermine que le passage de régénération (27A, 27B) est raccordé au passage côté veineux (22).

3. Appareil de dialyse (1) selon la revendication 1, **caractérisé en ce que**
le dispositif de détermination (5) calcule une pression différentielle entre une pression mesurée par le capteur de pression côté alimentation (S3) et une pression mesurée par le capteur de pression côté collecte (S4), dans un état où le liquide que distribue le dispositif de distribution de liquide (65) au circuit sanguin (3) à partir du passage de régénération (27A, 27B) est amené à s'écouler vers le passage de collecte de dialysat (55) via le passage de connexion (24A, 24B, 25) à partir d'une partie d'extrémité du passage côté veineux (22), et
lorsque la pression différentielle est inférieure à un deuxième seuil, le dispositif de détermination (5) détermine que le passage de régénération (27A, 27B) est raccordé au passage côté veineux (22), et lorsque la pression différentielle est supérieure au deuxième seuil, le dispositif de détermination (5) détermine que le passage de régénération (27A, 27B) est raccordé au passage côté artériel (21).

4. Procédé de vérification du raccordement d'un passage de régénération (27A, 27B) avant réalisation d'un traitement de dialyse et n'incluant pas de traitement de dialyse, incluant un dialyseur (2) qui réalise une hémodialyse, un passage d'alimentation en dialysat (54) qui fournit un dialysat frais au dialyseur (2), un passage de collecte de dialysat (55) qui collecte un dialysat usagé qui passe à travers le dialyseur (2), un circuit sanguin (3) comprenant un passage côté artériel (21) qui fournit du sang au dialyseur (2) et un passage côté veineux (22) qui évacue le sang du dialyseur (2), un passage de régénération (27A, 27B) prévu entre le passage d'alimentation en dialysat (54) et le circuit sanguin (3), et le dispositif de distribution de liquide (65) qui distribue le dialysat dans le passage d'alimentation en dialysat (54) au circuit sanguin (3) via le passage de régénération (27A, 27B), et le fait de déterminer si le passage de régénération (27A, 27B) est raccordé au passage côté artériel (21), ou au passage côté veineux (22) dans le circuit sanguin (3), le procédé étant **caractérisé en ce que**
un capteur de pression côté alimentation (S3) qui mesure une pression dans le passage d'alimentation en dialysat (54), et un capteur de pression côté collecte (S4) qui mesure une pression dans le passage de collecte de dialysat (55) sont prévus,
dans un état où l'un ou l'autre du passage côté artériel (21) ou du passage côté veineux (22) du circuit sanguin (3) est amené à communiquer avec le passage de collecte de dialysat (55), le dispositif de distribution de liquide (65) amène un liquide à s'écouler dans le circuit sanguin (3) à partir du passage de régénération (27A, 27B), et le liquide est amené à s'écouler vers le passage de collecte de dialysat (55) à partir du passage côté artériel (21) ou du passage côté veineux (22), et
il est déterminé si une destination de raccordement du passage de régénération (27A, 27B) est le passage côté artériel (21) ou le passage côté veineux (22) sur la base d'une pression différentielle entre une pression mesurée par le capteur de pression côté alimentation (S3) et une pression mesurée par le capteur de pression côté collecte (S4).

5. Procédé de vérification du raccordement d'un passage de régénération (27A, 27B) selon la revendication 4, **caractérisé en ce qu'**il comprend :
le calcul d'une pression différentielle entre une pression mesurée par le capteur de pression côté alimentation (S3) et une pression mesurée par le capteur de pression côté collecte (S4), dans un état où le liquide distribué au circuit sanguin (3) à partir du passage de régénération (27A, 27B) par le dispositif de distribution de liquide (65) est amené à s'écouler vers le passage de collecte de dialysat (55) via le passage de raccordement (24A, 24B, 25) à partir d'une partie d'extrémité du passage côté artériel (21), et
la détermination que le passage de régénération (27A, 27B) est raccordé au passage côté artériel (21) lorsque la pression différentielle est inférieure à un premier seuil, et la détermination que le passage de régénération (27A, 27B) est raccordé au passage côté veineux (22) lorsque la pression différentielle est supérieure au premier seuil.

6. Procédé de vérification d'un raccordement d'un passage de régénération (27A, 27B) selon la revendication 4, **caractérisé en ce qu'**il comprend :
le calcul d'une pression différentielle entre une pression mesurée par le capteur de pression côté alimentation (S3) et une pression mesurée par le capteur de pression côté collecte (S4) dans un état où le liquide distribué au circuit sanguin (3) à partir du passage de régénération (27A, 27B) par le dispositif de distribution de liquide (65) est amené à s'écouler vers le passage de collecte de dialysat (55) via le passage de raccordement (24A, 24B, 25) à partir d'une partie d'extrémité du passage côté veineux (22), et
la détermination que le passage de régénération (27A, 27B) est raccordé au passage côté veineux (22) lorsque la pression différentielle est inférieure à un deuxième seuil, et la détermination que le passage de régénération (27A, 27B) est raccordé au passage côté artériel (21) lorsque la pression différentielle est supérieure au deuxième seuil.
